# EUROPEAN PATENT SPECIFICATION

(11) **EP 2 341 061 B1**
(45) Date of publication and mention of the grant of the patent: **31.07.2013**
(21) Application number: 10197385.7
(22) Date of filing: 30.12.2010
(51) Int. Cl.: C07K 1/00, C07K 1/18, C07K 14/535

(54) **A novel process for preparing G-CSF (granulocyte colony stimulating factor)**
Neuartiges Verfahren zur Herstellung von G-CSF
Nouveau procédé de préparation du G-CSF (facteur de stimulation de colonie de granulocytes)

(30) Priority: 31.12.2009 TR 200910033
(43) Date of publication of application: 06.07.2011
(73) Proprietor: Sanovel Ilaç Sanayi Ve Ticaret Anonim Sirketi, 34460 Sariyer/Istanbul (TR)
(72) Inventor: Toksöz, Ahmet, 34398 Istanbul (TR); Yenice, Irem, 34398 Istanbul (TR); Üzgün, Mehmet, 34398 Istanbul (TR); Öner, Filiz, 34398 Istanbul (TR)
(74) Representative: Sevinç, Erkan

(56) References cited:
- EP-A1- 2 058 326
- WO-A2-2007/107882
- WO-A2-2008/096370
- ANONYMOUS: "NEUPOGEN - filgrastim injection, solution", DailyMed , 1 March 2007 (2007-03-01), XP002591618, Retrieved from the Internet: URL:http://dailymed.nlm.nih.gov/dailymed/a rchives/fdaDrugInfo.cfm?archiveid=10056 [retrieved on 2010-07-08]
- FISCHER B ET AL: "ISOLATION, RENATURATION, AND FORMATION OF DISULFIDE BONDS OF EUKARYOTIC PROTEINS EXPRESSED IN ESCHERICHIA COLI AS INCLUSION BODIES", BIOTECHNOLOGY AND BIOENGINEERING, WILEY & SONS, HOBOKEN, NJ, US LNKD- DOI:10.1002/BIT.260410103, vol. 41, no. 1, 5 January 1993 (1993-01-05), pages 3-13, XP000605146, ISSN: 0006-3592
- MUKHOPADHYAY A: "Inclusion bodies and purification of proteins in Biologically active forms", ADVANCES IN BIOCHEMICAL ENGINEERING, BIOTECHNOLOGY, SPRINGER, BERLIN, DE LNKD- DOI:10.1007/BFB0103030, vol. 56, 1 January 1997 (1997-01-01), pages 61-109, XP003023356, ISSN: 0724-6145
- GETZ E B ET AL: "A comparison between the sulfhydryl reductants tris(2-carboxyethyl)phosphine and dithiothreitol for use in protein biochemistry.", ANALYTICAL BIOCHEMISTRY 15 AUG 1999 LNKD- PUBMED:10452801, vol. 273, no. 1, 15 August 1999 (1999-08-15), pages 73-80, XP002634407, ISSN: 0003-2697
- RAO DASARI V K ET AL: "Optimization of the downstream process for high recovery of rhG-CSF from inclusion bodies expressed in Escherichia coli", PROCESS BIOCHEMISTRY, ELSEVIER, NL LNKD- DOI:10.1016/J.PROCBIO.2008.01.024, vol. 43, no. 5, 1 May 2008 (2008-05-01), pages 566-575, XP022588872, ISSN: 1359-5113 [retrieved on 2008-02-07]

## Description

### Technical Aspect

The present invention is related to a novel process of isolating and purifying granulocyte colony stimulating factor (G-CSF) from a G-CSF-producing microorganism, more specifically G-CSF is recombinant methionyl human G-CSF (rmetHuG-CSF).

### Background of the Invention

Granulocyte colony stimulating factor (G-CSF) is a 175 amino acid protein manufactured by recombinant DNA technology. G-CSF is produced by Escherichia coli (E coli) bacteria into which has been inserted the human granulocyte colony-stimulating factor gene. The protein has an amino acid sequence that is identical to the natural sequence predicted from human DNA sequence analysis, except for the addition of an N-terminal methionine necessary for expression in E coli. Because G-CSF is produced in E coli, the product is nonglycosylated and thus differs from G-CSF isolated from a human cell.

G-CSF is supplied in either vials or in prefilled syringes. The product is available in single use vials and prefilled syringes. The single use vials contain either 300 mcg or 480 mcg G-CSF at a fill volume of 1,0 ml or 1,6 ml respectively. The single use prefilled syringes contain either 300 mcg or 480 mcg G-CSF at a fill volume of 0,5 ml or 0,8 ml respectively.

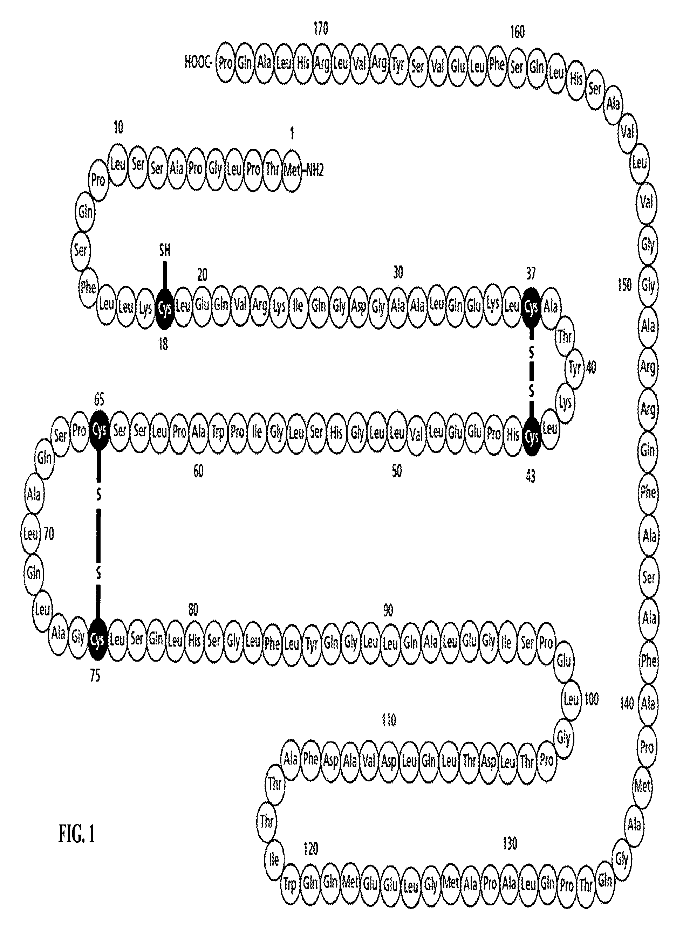

The amino acid sequence of human G-CSF is shown in **Figure 1**. The first amino acid of the mature protein was determined by direct amino acid sequencing of the purified proteins. Human G-CSF mRNAs code for a 30 amino acid hydrophobic leader sequence typical of secreted proteins. G-CSF has two disulphide bonds formed by homologous cysteine residues (Figure 1) and an extra cysteine residue at position at 17 that cannot participate in intramolecular disulfide bonds. The disulfide bonds in these molecules stabilize the structures and make them resistant to relatively harsh treatment (some proteases, high temperatures, denaturing solvents, extreme pH), which do lead to denaturation after reduction of disulfide bonds (Nicos A. Nicola, The walter and Eliza Hall Institute of Medical Research, "Granulocyte Colony Stimulating Factor", p. 77-100)

Human G-CSF can be produced in eukaryotic organisms (yeast and mammalian cell lines) and prokaryotic organism like bacteria. The form of G-CSF is produced depends on the type of host organism used for expression. If the G-CSF is expressed in eukaryotic cells, it is produced in a soluble form and secreted. When G-CSF is produced in prokaryotic cells, the product is formed as inactive inclusion bodies (IBs). Normally inclusion bodies have a secondary structure and are densely aggregated. It is revealed that the combination of so many factors of physiological state of host cell and growth conditions is affected by the formation of inclusion bodies.

G-CSF produced in procaryotic cells such as those of E.coli, is in inclusion bodies. Inclusion bodies are nuclear or cytoplasmic aggregates of stainable substances, usually proteins. The purification of the expressed proteins from inclusion bodies usually requires two main steps; extraction of inclusion bodies from the bacteria followed by the solubilisation of the purified inclusion bodies.

Production of biologically active human G-CSF protein from inactive inclusion bodies expressed by rDNA technology in commonly used prokaryotic host cells is very difficult. Efficient process methodologies are desirable for manufacture of therapeutically useful G-CSF on industrial scale. Various methods have been reported in scientific literature for the production of G-CSF in E.coli, yeast, or mammalian CHO (Chinese hamster ovary) cells.

Many patents describe various aspects of expression and purification of G-CSF protein from different expression systems like prokaryotic and eukaryotic cells. Expression of G-CSF in bacterial systems is described in United States patents, US 4 810 643 (03.03.1986, Kirin-Amgen Inc.) and US 4 999 291 (23.08.1985, Amgen Inc.). In the preferred processes, G-CSF gene is synthesized, cloned and transformed into in E.coli. The protein is expressed by inducing the culture by elevating the temperature up to 42°C. Conditions and parameters for cloning and expression by fermentation are not clear. A number of steps are involved in clone construction. The process is performed under flask conditions and the level of protein expression is 3 - 5% only on total cellular protein basis.

The pharmaceutical product called NUPOGEN®, a trademark of Amgen Inc., includes a 175 amino acid protein manufactured by recombinant DNA technology. The product information retreivable from the web site at:
http://dailvmed.nlm.nih.aov/dailymed/archives/fdaDrualnfo.cfm?archiveid=10056 discloses that NEUPOGEN® is produced by Escherichia coli (E coli) bacteria into which has been inserted the human granulocyte colony-stimulating factor gene. The formulation of the product contains Filgrastim, Acetate, Sorbitol, Polysorbate 80, Sodium and Water in a form suitable for intravenous and subcutaneous administration.

WO 2008/096370-A2 discloses a process for isolating and purifying G-CSF protein from a G-CSF producing E. coli comprising lysing the microorganism and separating G-CSF, isolating inclusion bodies comprising G-CSF, solubilizing G-CSF with a concentrated chaotropic agent and a reducing agent such as DTT, oxidising G-CSF, refolding - concentrating and desalting the same, subjecting it to ion exchange chromatography, and recovering purified G-CSF in a stable form.

GETZ E B ET AL: "A comparison between the sulfhydryl reductants tris(2-carboxyethyl)phosphine and dithiothreitol for use in protein biochemistry." Analytical Biochemistry 15 AUG 1999 LNKD-PUBMED: 10452801, vol. 273, no. 1, pages 73-80 presents a comparison between dithiothreitol (DTT) and tris(2-carboxyethyl)phosphine (TCEP) in terms of: (1) stability at neutral pH, (2) ability to preserve enzymatic activity, (3) interference with attachment of labels to protein thiols, (4) reduction of nitroxide spin probes, and (5) ability to cause unwanted protein degradation at elevated temperatures used in gel electrophoresis preparations. TCEP is however not proposed for increasing the process yield by efficiently breaking disulphide bonds with an unfolding activity in a procedure, for instance for production of granulocyte colony stimulating factor (G-CSF).

EP 0 220 520 (30.09.1985, Chugai Seiyaku Kabushiki Kaisha), WO 87/01132 (23.08.1985, Kirin-Amgen Inc.) and WO 88/01297 (11.08.1986, Cetus Corporation) describe the cloning of G-CSF.

United States patent, US 5 055 555 (05.01.1989, Sassenfeld, Helmut) describes a simplified process for production of human G-CSF from eukaryotic cells. Achieving protein secretion through extracellular eukaryotic organisms forms an entirely different technology in comparison with protein expression through intracellular prokaryotic organisms. Although the process described in patent US 5,055, 555 is simpler; it is not readily applicable to recombinant G-CSF expressed in E. coli or other cells where G-CSF is produced in the form of an inclusion body. The above method was developed for soluble G-CSF expressed and secreted by recombinant yeast into the fermentation medium. For E. coli derived G-CSF, solubilization of inclusion bodies and refolding of G-CSF are additional steps to be taken into account. Besides, obtaining a therapeutic grade preparation of G-CSF free of lipopolysaccharide endotoxins, which are likely contaminants when E. coli is used as a host, in a simple, scaleable procedure has not been reported so far. On a commercial scale, yield losses from a multi-step process become highly significant. Hence a simplified procedure with fewer steps will give higher yields in a shorter time, besides being economical.

The production of recombinant therapeutic proteins using Escherichia coli as host system is viable if one can set up a simple and cost-effective downstream process. The high-level expression of eukaryotic proteins in E. coli often leads to formation of insoluble lBs in the cytoplasm. IBs formation may occur as a consequence of intracellular accumulation of partially unfolded forms of the recombinant protein. Protein properties such as the average charge, turn-forming residue fraction, cysteine and proline fractions, hydrophilicity, and total number of residues have been correlated with inclusion body formation. Culture conditions such as temperature, pH, and nutrient supply play a vital role in controlling the partition of the recombinant protein into soluble and insoluble fractions. It is often difficult to recover the protein from lBs due to the problems associated with the initial recovery, solubilization and renaturation steps.

The various purification protocols discussed in the above patents mention multiple chromatography and other steps for the purification of G-CSF. None of the above-related patents disclose a simpler, economical and commercially method for the production of G-CSF at a commercially viable scale which guarantees the stability of the product obtained by the process. The processes described in prior art are complex, lengthy and unit costs are high.

Therefore, to overcome the major problems associated with the manufacture of G-CSF, a simple, economical, industrially applicable and more stable process for high recovery of G-CSF has been now developed and disclosed in this present invention.

### Summary of the Invention

The present invention relates to a novel process of isolating and purifying granulocyte colony stimulating factor (G-CSF) from a G-CSF-producing microorganism and describes an industrially applicable, simple, cost-effective, time-saving method of producing granulocyte colony stimulating factor (G-CSF).

The main object of the present invention is to provide a novel process for isolating and purifying granulocyte colony stimulating factor (G-CSF) from a G-CSF-producing microorganism consisting the following steps;
a) lysing the microorganism by adjusting density and temperature of cell paste suspension and separating insoluble material comprising G-CSF,
b) isolating the inclusion bodies (IBs) comprising G-CSF in the presence of ionic surfactant agent
c) solubilising the G-CSF present in the insoluble material, using a high concentrated chaotropic agent and a denaturing agent which is tris(2-carboxyethyl)phosphine HCl (TCEP)
d) oxidizing the G-CSF in the presence of cystine/cysteine wherein the molar ratio is 1:10,
e) refolding the G-CSF in temperature controlled conditions by a one step method,
f) concentrating the refolded G-CSF solution by using Tangential Flow Filtration (TFF) system using 5-12 KDa molecular weight cut off cassettes or cartusches,
g) desalting of concentrated G-CSF solution in a temperature controlled column and separating the G-CSF solution from chaotrope by gel-filtration,
h) subjecting the G-CSF solution to cation exchange chromatography,
i) recovering purified G-CSF in formulation buffer in the stable form,
j) removing endotoxin by using strong-basic anion exchange chromatography.

In one embodiment, the G-CSF is recombinant methionyl human G-CSF (rmetHuG-CSF).

In one embodiment of the invention, the cell paste density of lysing step is not less than 8 mL of resuspension buffer per gram IB and the temperature is in between 5 to 20 °C, preferably it is in between 5 to15 °C.

Another embodiment of the invention is the ionic surfactant agent wherein it is sodium deoxycholate in the isolating step.

In one embodiment, the high concentrated chaotropic agent in the solubilising step is guanidinium HCl in the concentrations of 5.4 M to 6.6 M, preferably it is 6M.

In one embodiment, the temperature in the refolding step is between 7 to 15 °C, preferably it is between 7 to 11°C, more preferably it is 8 to 10°C.

According to another embodiment, the gel-filtration column is Sephadex G-25 Fine and the particle size of Sephadex G25 Fine beads is between 20 and 300 µm, preferably it is about 80 µm.

In one embodiment, the column in desalting step is stabilized in a temperature between 4 to 20 °C, preferably it is between 7 to 15 °C, more preferably it is between 7 to 13 °C.

In one embodiment, the cation exchange chromatography consists of sulfopropyl and the stable formulation buffer in the recovering step is an aqueous solution of 10 mM Acetate Buffer (or sodium acetate), %5 Sorbitol and %0.004 Polysorbate 80 which is adjusted to pH 4.0.

In another embodiment, the strong-basic anion exchange chromatography step used in removing endotoxins is consisting of quaternary ammonium.

In one embodiment the pH in step c) is from 7.5 to 9.0, preferably it is 8. and In another embodiment the pH in step e) is from 7.0 to 8.0, preferably it is 7.5.

According to another embodiment, the microorganism producing G-CSF is E.coli.

In a further embodiment of the invention, the method comprises the further step of formulating the rmetHuG-CSF **for producind a** biopharmaceutical product consisting of;
a) 30-100 MU (300 - 1000 µg) rmetHuG-CSF
b) 0,50 - 0.80 mg of acetate (or 0,250 - 1,00 mg of sodium acetate)
c) 25 - 100 mg sorbitol
d) 0,01 - 0,10 mg polysorbate 80
e) water for injection
f) sodium hydroxide **or acetic acid for pH adjustment**

in 1 ml sterile solution, which is acceptable for filling into appropriate delivering devices of defined dosage strengths for administration to patients.

In a preferred embodiment the amount of acetate is 0.59 mg.

**B**iopharmaceutical product**s** obtained by using the process of the present invention **can be**, useful in the treatment of neutropenia, leukaemia, mobilization of peripheral blood progenitor cells (PBPC), HIV infections such as bacterial infections and cancer patients receiving cytotoxic chemotarapy, myelosuppressive chemotherapy and bone marrow transplant

In prior art, different type of the microorganisms, different type of strain and also different modified gene sequences to produce of the G-CSF are described. Each process has specific production conditions and their problems that can be obtained from the process conditions or produced target protein or impurities. Development of the purification steps in protein production depends on the un-purified protein specifications obtained from up-stream process in different conditions. The present invention is related to production of the pure and stable G-CSF (%98 according to "size exclusion method" of EP) as a final product considering of the time, labor and cost consuming. The processes available to date are generally time, labor and cost consuming as well as being complicated. Moreover, they fail to provide stable results. There is still a need for improved purifications results.

### Detailed Description of the invention

A simple and innovative method for the production of G-CSF which is recombinant methionyl human G-CSF (rmetHuG-CSF) is carried out by using E.coli cells due to the high level of protein expression and avoidance of glycosylation complications.

E.coli cells are harvested after fermentation by centrifugation and the proteinaceous material, which is in the form of inclusion body, is extracted from cells by lysing the bacterial cells using a high pressure cell disrupter by adjusting density and temperature of cell paste suspension and separating insoluble material comprising G-CSF. Bacterial cell wall is broken by physical means thus enabling inclusion bodies in the cells to suspend freely in buffer. Inclusion bodies that are suspended in the buffer solution are sedimented by high speed centrifugation. Inclusion body comprising G-CSF is isolated in the presence of ionic surfactant agent. After washing and centrifugation steps, inclusion bodies are solubilized in a suitable solubilization buffer containing a high concentrated choatropic agent. The solubilization buffer is aqueous solution with pH value of 8.0 comprising 6M Guanidine.HCl as chaotropic agent and 100mM TRIS as buffering salt. Inclusion bodies are solubilized with the buffer mentioned above at room temperature.

### Purification

After solubilization step of inclusion bodies, there are several different proteinaceous materials, not only different from G-CSF but also several isoforms of G-CSF. There is a need to change the conformation of misfolded G-CSF and break intermolecular disulphide bridges which may form dimers, oligomers and aggregates. Thus, solubilized proteins with a proper denaturing agent are denatured to obtain unfolded monomer chains. The denaturation step of the proteins is important and it affects the final product yield and purity. If misfolded protein remains in the solution it may trigger aggregation trough all purification steps. Thus 0.5M tris-2-carboxyethylphoshpine.HCl (TCEP) or 0.5M DTT is chosen as a strong denaturing agent to obtain desired denaturation levels. In 1-2 hours of mixing at room temperature enables proper levels of denaturation.

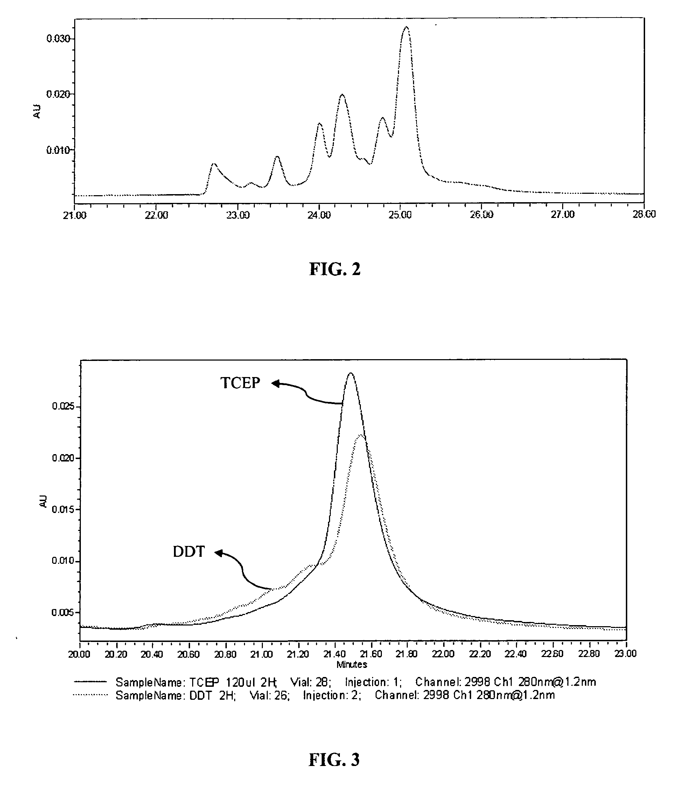

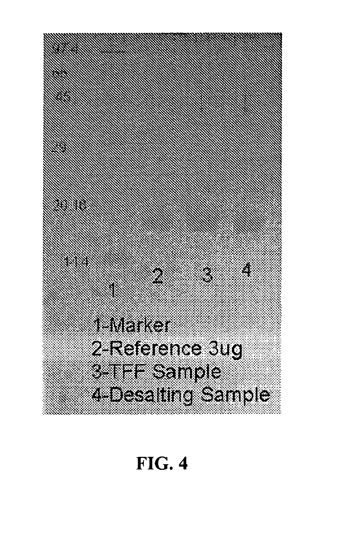

DTT is an odorous chemical and when it contacts with the air, it can be easily oxidised. Due to air oxidation it is difficult to store DDT as a solvent and it is relatively unstable compound. Thus, TCEP is chosen for the present invention. Surprisingly, it is found that it has a synergistic effect over unfolding of the protein which is shown in Figure 3. As a result of this effect the yield is increased at the end of the process.

Figure 2 shows the chromatogram of the inclusion bodies in the solution buffer. In this chromatogram it is seen that all the proteins are in a mixture including the G-CSF because of the inclusion bodies.

Figure 3 shows the chromatogram after adding the TCEP and DDT to the solubilisation buffer and the effect of TCEP is clearly seen in unfolding misfolded GCSF with different conformations. In Fig. 3, DDT peak is the one below 0.025 AU and TCEP peak is the one over 0.025 AU.

It is clearly shown that the peak which refers to TCEP have a higher peak and a more flat and smooth surface without extra peaks than the peak refers to DDT. DDT peak has also 3 little peaks which show that it is not completely successful to obtain unfolded protein in a flat **chain**. This shows the synergistic effect of the TCEP over the unfolding step and the total protein concentration is increased by 10-15 %

Refolding of G-CSF molecules is conducted in the presence of a pair of oxidizing/reducing agent. Cystine/cysteine couple is used to obtain properly folded G-CSF molecule formation. The molar ratio of cystine to cysteine is 1/10. Refolding solution is aqueous solution with pH value 7.5 comprising 0.5 M L-Arginine.HCl, 50 mg/ml Sorbitol, 0.1M TRIS and 2mM EDTA. Refolding solution volume is adjusted to reach 0.2mg/ml concentration of denatured protein. The solution is kept in between 7 to 15°C, preferably in between 7 to 11°C, more preferably 8 to 10°C for 16-20 hours to reach proper folding conformation and desired folding levels.

When folding of proper conformation is finalized, there is need for concentrating the solution to smaller volumes for further purification steps which involves chromatography. Tangential Flow Filtration (TFF) is used to remove undesired molecules which have molecular weight smaller than 5-12 kDa. By this way, not only solution is concentrated but also some process related impurities such as small protein molecules which could not removed in previous steps could be removed.

The concentration of the protein effects the concentration of the finalised product concentration and accordingly the yield of the process. So the use of TFF is very important in this step.

The concentrated solution is then subjected to gel filtration so that the buffer salts are removed along with small molecular weight proteinaceous material. The protein is separated from said salt by the huge molecular size difference by injection into and moving in the desalting column. Desalting column is packed with Sephadex G-25 Gel which has a bead size in between 20 - 300 µm, preferably about 80 µm, at which resolution of G-CSF with other proteinaceous material is better, thus giving more pure intermediate product in terms of process impurities. The desalting column is cooled to 4 - 20°C so that the concentrated sample in the column outlet stays stable. The concentrated solution of TFF step is injected to column and moved with a mobile phase is pH=5.4 with conductivity value 1.0 - 2.0 uS/cm comprising 20 mM Sodium Asetate and 50 mg/ml Sorbitol.

The outlet solution of desalting is then subjected to cation exchange chromatography. This is the step in which properly folded G-CSF is removed from its misfolded isoforms and other impurities. The proteins are first fed to a cation exchange column filled with strong cation exchange material and they are hold by the media. Then gradually using a gradient method, the conductivity is increased to certain values enabling the impurities and G-CSF to elute on different times for separation with respect to their different pl values. Gradient method utilizes usage of two different mobile phases of two different conductivity values: mobile phase A and mobile phase B. Mobile phase A is a pH=5.4 solution, having conductivity value 1.0 - 2.0 uS/cm, comprising 20mM Sodium Asetate and 50mg/ml Sorbitol. Mobile phase B is a pH=5.4 solution, having conductivity value 40-50 uS/cm, comprising 20mM Sodium Asetate, 50mg/ml Sorbitol and 0.5M NaCl.

Pure protein material present in the buffered water solution is the subjected to gel filtration to produce the final product. Thus, active G-CSF molecule is directly recovered as final product with a proper and stable formulation avoiding complexity and problems of other recovery techniques, such as lyophilization and reconstitution. Gel filtration is carried out with the same matrix in desalting step but with different mobile phase composition, which is named as formulation buffer. It comprises 10mM acetate buffer and 50mg/ml Sorbitol and %0.004 Polysorbate 80 with pH value 4.0

Endotoxin level is essential in producing sterile injectable solution such as the product which is produced by present invention. In production biological and biotechnological active substances, sterile solutions such as washing buffers, mobile phases and formulation solutions can be very costly which may also require a controlled environment that will also contribute to high cost by its requirement for regular microbiological testing and control. It is a challenge to keep endotoxin levels if a fully controlled sterile environment is not available. The present invention allows the production costs drop significantly by making it possible to work in a non-sterile environment and keep the endotoxin levels comply with EP monograph. The present invention utilizes ion exchange chromatography in which ion exchange media is a strong basic anion exchanger. The strong anion exchange media can be quaternary ammonium. Ion exchange chromatography is the most common depyrogenation method for proteins; however, it has several drawbacks, which limit its usefulness as depyrogenation step. This includes handling and usage problems such as packing, channeling, low flow rates, long regeneration times, compressibility and limited chemical stability. Small flow rates and susceptibility to fouling mean that incorporating chromatography resins into process scale purification steps can be expensive and troublesome. A high capacity, scaleable and ready-to-use ion exchange membrane technology provides depyrogenation in process. By utilization of ion exchange chromatography with strong basic anion exchangers, the endotoxin levels can be dropped much below.

As described detailed above the sequence of the purification step is important according to develop a pure, simple, effective and commercially economical and more stable process for high-level expression of G-CSF.

Cation exchange step is performed following the desalting step which allows removing of high salt ingredients.

The importance of the sequence is also shown by the chromatografic data in below Figure 3 by SDS-PAGE analyse. Marked samples with the arrow, in 3^{rd} and 4^{th} row between 29 and 45 kDa in reference marker are the ones which are removed by cation exchange step.

In a preferred embodiment of the invention, biologically active G-CSF obtained by using the process of the present invention can be used for preparation of medicaments.

According to this embodiment of the invention, one of the medicament is the biopharmaceutical product manufactured according to process of the described invention is consisting of
a) 30-100 MU (300 - 1000 µg) of rmetHuG-CSF
b) 0.50 - 0.80 mg of acetate, preferably 0.59 mg of acetate
c) 25 - 100 mg of sorbitol
d) 0,01 - 0,10 mg of polysorbate 80
e) water for injection
f) pH is adjusted with sodium hydroxide
in 1 ml sterile solution.

Such medicaments are indicated for a wide range of indications and include; neutropenia and neutropenia-related clinical sequelae, chronic neutropenia, neutropenic and non-neutropenic infections, reduction of hospitalisation for febrile neutropenia after cytotoxic chemotherapy and for the reduction in the duration of neutropenia in patients undergoing myeloablative therapy followed by bone marrow transplantation considered to be at increased risk of prolonged severe neutropenia.

G-CSF is also indicated for mobilisation of peripheral blood progenitor cells (PBPC) and chronic inflammatory conditions.

Its long term administration is indicated to increase neutrophil counts and to reduce the incidence and duration of infection-related events, treatment of persistent neutropenia in patients with advanced HIV infection, in order to reduce the risk of bacterial infections.

G-CSF is also indicated for improving the clinical outcome in intensive care unit patients and critically ill patients, wound/skin ulcers/burns healing and treatment, intensification of chemotherapy and/or radiotherapy, increase of anti-inflammatory citokines, potentiation of the antitumour effects of photodynamic therapy.

It is indicated for prevention and treatment of illness caused by different cerebral disfunctions, treatment of thrombotic illness and their complications and post irradiation recovery of erythropoiesis. It can be also used for treatment of all other illnesses, which are indicative for G-CSF.

A biopharmaceutical composition containing the pure and biologically active G-CSF obtained by the process of the invention can thus be administered, to patients, children or adults in a therapeutical amount which is effective to treat the above mentioned diseases.

The present invention is further described by referring to the following example. Although the example is not intended to limit the scope of the present invention, it should be considered in the light of the description detailed above.

### Example

### Desalting

G-CSF is separated from chaotropic agents and oxidizing/reducing pair as well as other process related impurities and buffer salts of solubilization and refolding step. At this step the intermediate product is a concentrate from TFF step comprising salts which need to be separated from G-CSF and removed. The mobile phase for this step is an aqueous solution of pH=5.4 with conductivity value 1.0 - 2.0 uS/cm comprising 20mM Sodium Acetate and 50mg/ml Sorbitol. As the sample is injected into column, the absorbance and conductivity value of the outlet solution is carefully monitored at 280 nm wavelength for absorbance and about 1.35 umS/cm for conductivity. The baseline for each parameter is maintained until molecules are eluted. Since protein molecules are eluted first, the first observed peak is on absorbance baseline which corresponds to that the protein coming out of the column. Product is separated until the first peak is eluted. Then other proteinaceous material with smaller molecules of G-CSF gives another UV peak eluting just before an increase in conductivity which correspond to elution of salts.

### Cation Exchange

Properly folded G-CSF is removed from its impurities and purified. Intermediate product is taken after desalting step and fed to cation exchange column with mobile phase A which is pH=5.4, having conductivity value 1.0 - 2.0 uS/cm, comprising 20mM Sodium Asetate and 50mg/ml Sorbitol. While sample is being fed to column, 280nm UV baseline is maintained. The increase in baseline is the signal of column holding capacity. Proper column volume should be chosen to avoid column saturation. After sample is fed, purification process continues with gradual mobile phase B injection into column with a gradient program to allow protein separation and impurities. Mobile phase B is pH=5.4, having conductivity value 40 - 50 uS/cm, comprising 20mM Sodium Asetate, 50mg/ml Sorbitol and 0.5M NaCl. Its higher conductivity value enables change of salt concentration in the column to separate proteins.

### Gel Filtration for Final Step

G-CSF is recovered directly into the formulation buffer. At this step the intermediate product is in the cation exchange outlet buffer which contains 0.1 - 0.5 M NaCl. Since the product is IV injection the salt and the product should be separated thus gel filtration chromatography is conducted. As mentioned above, the mobile phase for this step is the formulation buffer which is aqueous solution with pH value 4.0, comprising 10mM Acetate buffer and 50mg/ml Sorbitol and %0.004 Polysorbate 80. As the sample is injected into column, the absorbance and conductivity value of the outlet solution is carefully monitored at 280 nm wavelength for absorbance and about 1.35 umS/cm for conductivity. The baseline for each parameter is maintained until molecules are eluted. Since protein molecules are eluted first, the first observed peak is on absorbance baseline which corresponds to that the protein coming out of the column. Product is separated until an increase in conductivity is monitored which corresponds to elution of salts are taking place.

### Ion Exchange

This is the optional step in which G-CSF containing product could be further removed of its endotoxin impurities. At this step final product which is G-CSF recovered to formulation buffer as in gel filtration step, is fed to ion exchanger column continuously in a recycling manner for a number of times and at the end endotoxin free sample is sterile filtered for filling.

## Claims

1. A process for isolating and purifying granulocyte colony stimulating factor (G-CSF) from a G-CSF-producing microorganism consisting of the following steps;
a) lysing the microorganism by adjusting density and temperature of cell paste suspension and separating insoluble material comprising G-CSF,
b) isolating the inclusion body (IB) comprising G-CSF in the presence of ionic surfactant agent
c) solubilising the G-CSF present in the insoluble material, using a high concentrated chaotropic agent and a denaturing agent which is tris(2-carboxyethyl)phosphine HCl (TCEP)
d) oxidizing the G-CSF in the presence of cystine/cysteine wherein the molar ratio is 1:10,
e) refolding the G-CSF in temperature controlled conditions by a one step method,
f) concentrating the refolded G-CSF solution by using Tangential Flow Filtration (TFF) system using 5-12 KDa molecular weight cut off cassettes or cartusches,
g) desalting of concentrated G-CSF solution in a temperature controlled column and separating the G-CSF solution from chaotrope by gel-filtration,
h) subjecting the G-CSF solution to cation exchange chromatography,
i) recovering purified G-CSF in formulation buffer in the stable form,
j) removing endotoxin by using strong-basic anion exchange chromatography.

2. The process according to claim 1, wherein the G-CSF is recombinant methionyl human G-CSF (rmetHuG-CSF).

3. The process according to claim 1 and 2, wherein the cell paste density is not less than 8ml of resuspension buffer per gram IB and temperature is in between 5 to 20°C.

4. The process according to claim 1 and 2, wherein the ionic surfactant agent in the isolating step is sodium deoxycholate.

5. The process according to claims 1 and 2, wherein the high concentrated chaotropic agent in the solubilising step is guanidinium HCl in the concentrations between 5.4 M to 6.6 M.

6. The process according to claims 1 and 2, wherein the temperature in the refolding step is between 7 to 15 °C.

7. The process according to claims 1 and 2, wherein the gel-filtration column is Sephadex G-25 Fine.

8. The process according to claim 7, wherein the particle size of Sephadex G25 Fine beads is between 20 and 300 µm.

9. The process according to claims 1 and 2, wherein the column is stabilized in a temperature between 4 to 20 °C degrees in desalting step.

10. The process according to claims 1 and 2, wherein the cation exchange chromatography is consisting of sulfopropyl.

11. The process according to claims 1 and 2, wherein the stable formulation buffer in the recovering step is an aqueous solution of 10 mM Acetate buffer, %5 Sorbitol and %0.004 Polysorbate 80 adjusted to pH 4.0.

12. The process according to claims 1 and 2, wherein the strong-basic anion exchange chromatography step used in removing the endotoxins is consisting of quaternary ammonium.

13. The process according to claims 1 and 2, wherein in step c) the pH is from 7.5 to 9.0.

14. The process according to claims 1 and 2 wherein in step e) the pH is from 7.0 to 8.0.

15. The process according to any of the preceding claims, wherein the microorganism producing G-CSF is E.coli.

16. The process according to claim 2, further comprising the step of formulating the rmetHuG-CSF according to a composition consisting of:
a) 30-100 MU (300 - 1000 µg) of rmetHuG-CSF
b) 0.50 - 0.80 mg of acetate
c) 25-100 mg of sorbitol
d) 0,01 -0,10 mg of polysorbate 80
e) water for injection
f) sodium hydroxide or acetic acid for pH adjustment
in 1 ml sterile solution.

## Patentansprüche

1. Verfahren zur Isolierung und Reinigung von Granulozyten-Kolonie-stimulierendem Faktor (granulocyte colony stimulating factor, G-CSF) aus einem G-CSF-produzierenden Mikroorganismus, bestehend aus den folgenden Schritten;
a) Lysieren des Mikroorganismus durch Einstellen von Dichte und Temperatur einer Zellpasten-Suspension (cell paste suspension) und Abtrennen unlöslichen Materials, das G-CSF umfasst,
b) Isolieren des Einschlusskörpers (inclusion body, IB), der G-CSF umfasst, in Gegenwart von ionischem oberflächenaktivem Mittel,
c) Solubilisieren des im unlöslichen Material vorhandenen G-CSF unter Verwendung eines hochkonzentrierten chaotropen Mittels und eines Denaturierungsmittels, das Tris(2-carboxyethyl)phosphin HCl (TCEP) ist,
d) Oxidieren des G-CSF in Gegenwart von Cystin/Cystein, wobei das molare Verhältnis 1:10 ist,
e) Rückfalten des G-CSF unter temperatur-kontrollierten Bedingungen durch ein Ein-Schritt-Verfahren,
f) Konzentrieren der rückgefalteten G-CSF-Lösung mit Hilfe eines Tangential-Fluss-Filtrations(Tangential Flow Filtration, TFF)-Systems, unter Verwendung von Kassetten oder Kartuschen mit einer Molekulargewichtsgrenze von 5-12 KDa (5-12 KDa molecular weight cut off),
g) Entsalzen der konzentrierten G-CSF-Lösung in einer temperatur-kontrollierten Säule und Abtrennen der G-CSF-Lösung vom Chaotrop durch Gelfiltration,
h) Durchführen einer Kationenaustauschchromatographie mit der G-CSF-Lösung,
i) Gewinnen von gereinigtem G-CSF in Formulierungspuffer in der stabilen Form,
j) Entfernen von Endotoxin durch Einsatz von stark basischer Anionenaustauschchromotographie.

2. Verfahren nach Anspruch 1, wobei das G-CSF rekombinantes Methionyl-human-G-CSF (recombinant methionyl human G-CSF, rmetHuG-CSF) ist.

3. Verfahren nach Anspruch 1 und 2, wobei die Zellpasten-Dichte nicht weniger als 8ml Resuspensionspuffer pro Gramm IB und die Temperatur zwischen 5 bis 20°C ist.

4. Verfahren nach Anspruch 1 und 2, wobei das ionische oberflächen-aktive Mittel beim Isolierungsschritt Natriumdeoxycholat ist.

5. Verfahren nach den Ansprüche 1 und 2, wobei das hochkonzentrierte chaotrope Mittel beim Solubilisierungsschritt Guanidinium HCl in Konzentrationen zwischen 5,4 M bis 6,6 M ist.

6. Verfahren nach den Ansprüchen 1 und 2, wobei die Temperatur beim Rückfaltungsschritt zwischen 7 bis 15°C ist.

7. Verfahren nach den Ansprüchen 1 und 2, wobei die Gelfiltrations-Säule Sephadex G-25 Fine ist.

8. Verfahren nach Anspruch 7, wobei die Teilchengröße von Sephadex G25 Fine-Perlen zwischen 20 und 300 µm liegt.

9. Verfahren nach den Ansprüchen 1 und 2, wobei die Säule beim Entsalzungsschritt bei einer Temperatur zwischen 4 bis 20°C stabilisiert ist.

10. Verfahren nach den Ansprüchen 1 und 2, wobei die Kationenaustauschchromatrographie aus Sulfopropyl besteht.

11. Verfahren nach den Ansprüchen 1 und 2, wobei der stabile Formulierungspuffer beim Gewinnungsschritt eine wässrige Lösung von 10 mM Acetatpuffer, 5% Sorbitol und 0,004% Polysorbat 80, eingestellt auf pH 4,0, ist.

12. Verfahren nach den Ansprüchen 1 und 2, wobei der stark basische Anionenaustauschchromatrographieschritt, der beim Entfernen der Endotoxine verwendet wird, aus quaternärem Ammonium besteht.

13. Verfahren nach den Ansprüchen 1 und 2, wobei bei Schritt c) der pH von 7,5 bis 9,0 ist

14. Verfahren nach den Ansprüchen 1 und 2, wobei bei Schritt e) der pH von 7,0 bis 8,0 ist.

15. Verfahren nach einem der vorangehenden Ansprüche, wobei der G-CSFproduzierende Mikroorganismus E.coli ist.

16. Verfahren nach Anspruch 2, ferner umfassend den Schritt zur Formulierung des rmetHuG-CSF gemäß einer Zusammensetzung bestehend aus:
a) 30 - 100 MU (300 - 1000 µg) rmetHuG-CSF
b) 0,50 - 0,80 mg Acetat
c) 25 - 100 mg Sorbitol
d) 0,01 - 0,10 mg Polysorbat 80
e) Wasser zur Injektion
f) Natriumhydroxid oder Essigsäure zur pH-Einstellung
in 1 ml steriler Lösung.

## Revendications

1. Procédé pour isoler et purifier le facteur de croissance des colonies granulocytes (G-CSF) à partir d'un microorganisme produisant le G-CSF constitué des étapes suivantes :
a) la lyse du microorganisme en ajustant la densité et la température d'une suspension de pâte cellulaire et en séparant le matériau insoluble comprenant le G-CSF,
b) l'isolement du corps d'inclusion (CI) comprenant le G-CSF en présence d'un agent tensioactif ionique,
c) la solubilisation du G-CSF présent dans le matériau insoluble, en utilisant un agent chaotropique fortement concentré et un agent dénaturant qui est la tris(2-carboxyéthyl)phosphine HCl (TCEP)
d) l'oxydation du G-CSF en présence de cystine/cystéine dans laquelle le rapport molaire est de 1 : 10,
e) le repliement du G-CSF dans des conditions de température contrôlée par un procédé à une étape,
f) la concentration du G-CSF replié en utilisant un système de filtration à flux tangentiel (FFT) en utilisant des cassettes ou des cartouches à tamis moléculaire de masse moléculaire de 5 à 12 kDa,
g) le dessalement de la solution de G-CSF concentrée dans une colonne à température contrôlée et la séparation de la solution de G-CSF du chaotrope par filtration sur gel,
h) la soumission de la solution de G-CSF à une chromatographie échangeuse de cations,
i) la récupération du G-CSF purifié dans la préparation tampon de forme stable,
j) l'élimination de l'endotoxine en utilisant une chromatographie d'échange d'anions fortement basiques.

2. Procédé selon la revendication 1, dans lequel le G-CSF est le méthionyl G-CSF humain recombinant (metHuG-CSF).

3. Procédé selon la revendication 1 et 2, dans lequel la densité de pâte n'est pas de moins de 8 ml de tampon en resuspension par gramme de CI et la température est comprise entre 5 et 20°C.

4. Procédé selon la revendication 1 et 2, dans lequel l'agent tensioactif ionique dans l'étape d'isolement est le désoxycholate de sodium.

5. Procédé selon les revendications 1 et 2, dans lequel l'agent chaotropique fortement concentré dans l'étape de solubilisation est le guanidium HCl aux concentrations entre 5,4 et 6,6 M.

6. Procédé selon les revendications 1 et 2, dans lequel la température dans l'étape de repliement est comprise entre 7 et 15°C.

7. Procédé selon les revendications 1 et 2, dans lequel la colonne de filtration sur gel est le Sephadex G25 Fine.

8. Procédé selon la revendication 7, dans lequel la taille de particule des billes de Sephadex G25 Fine est comprise entre 20 et 300 mm.

9. Procédé selon les revendications 1 et 2, dans lequel la colonne est stabilisée à une température entre 4 et 20°C degrés dans l'étape de dessalement.

10. Procédé selon les revendications 1 et 2, dans lequel la chromatographie échangeuse de cations est constituée de sulfopropyle.

11. Procédé selon les revendications 1 et 2, dans lequel la préparation tampon de formulation stable dans l'étape de récupération est une solution aqueuse de 10 mM de tampon acétate, sorbitol 5 % et polysorbate 80 0,004 % ajustée à pH 4,0.

12. Procédé selon les revendications 1 et 2, dans lequel l'étape de chromatographie échangeuse d'anions fortement basiques utilisée dans l'élimination des endotoxines est constituée d'ammonium quaternaire.

13. Procédé selon les revendications 1 et 2, dans lequel dans l'étape c) le pH est compris entre 7,5 et 9,0.

14. Procédé selon les revendications 1 et 2 dans lequel dans l'étape e) le pH est compris entre 7,0 et 8,0.

15. Procédé selon l'une quelconque des revendications précédentes, dans lequel le microorganisme produisant le G-CSF est *E coli.*

16. Procédé selon la revendication 2, comprenant en outre l'étape de formulation du metHuG-CSF selon une composition constituée de :
a) 30 - 100 MU (300 - 1000 mg) de metHuG-CSF
b) 0,50 - 0,80 mg d'acétate
c) 25 - 100 mg de sorbitol
d) 0,01 à 0,10 mg de polysorbate 80
e) eau pour injection
f) hydroxyde de sodium ou acide acétique pour l'ajustement du pH dans une solution stérile de 1 ml.
